(19) Europäisches Patentamt

European Patent Office

Office européen des brevets

(11) Veröffentlichungsnummer: **0 307 737 B1**

(12) **EUROPÄISCHE PATENTSCHRIFT**

(45) Veröffentlichungstag der Patentschrift: **25.11.92**

(21) Anmeldenummer: **88114392.9**

(22) Anmeldetag: **03.09.88**

(51) Int. Cl.⁵: **B01D 61/14**, C12M 1/12

(54) **Verfahren zur Abtrennung von biotechnologisch hergestellten Wertstoffen aus einer Fermenterbrühe durch Ouerstrom-Mikro-und/oder Ultrafiltration.**

(30) Priorität: **15.09.87 DE 3730868**

(43) Veröffentlichungstag der Anmeldung:
**22.03.89 Patentblatt 89/12**

(45) Bekanntmachung des Hinweises auf die
Patenterteilung:
**25.11.92 Patentblatt 92/48**

(84) Benannte Vertragsstaaten:
**AT BE CH DE FR GB LI NL**

(56) Entgegenhaltungen:
**EP-A- 0 137 877**
**EP-A- 0 200 032**
**DE-A- 3 531 836**
**DE-A- 3 716 363**
**FR-A- 2 295 777**

**PATENT ABSTRACTS OF JAPAN, vol. 9, no.
32 (C-265)[1755], 9. Februar 1985 & JP-A-59
179 110 (NITTO DENKI KOGYO K.K.)
11-10-1984**

**CHEM-ING.-TECH. 57 (1985), Nr. 9, Weinheim,
W. RÄHSE/F.-J. CARDUCK "Mikrofiltration von
Fermenterbrühen", Seiten 747-753**

(73) Patentinhaber: **Henkel Kommanditgesellschaft
auf Aktien
Postfach 1100 Henkelstrasse 67
W-4000 Düsseldorf-Holthausen(DE)**

(72) Erfinder: **Kühne, Norbert
Dürerstrasse 63
W-5657 Haan 1(DE)**
Erfinder: **Rähse, Wilfried, Dr. Dipl.-Ing.
Kolhagenstrasse 44
W-4000 Düsseldorf 13(DE)**
Erfinder: **Carduck, Franz-Josef, Dr.
Dipl.-Chem.
Landstrasse 18
W-5657 Haan(DE)**

(74) Vertreter: **Patentanwälte Meinke und Dabringhaus Dipl.-Ing. J. Meinke Dipl.-Ing. W. Dabringhaus
Westenhellweg 67
W-4600 Dortmund 1(DE)**

# Beschreibung

Die Erfindung richtet sich darauf, biotechnologisch hergestellte Wertstoffe aus einer Fermenterbrühe durch Querstrommikrofiltration in Wertstoffe und Rückstände zu trennen, wobei die Wertstoffe durch die Membran in das Permeat gelangen.

Derartige Verfahren zur Gewinnung von Wertstoffen aus Fermenterlösungen sind bekannt. In einer Querstrom-Membranfiltration wird der gelöste Wertstoff als Permeat aus der Fermenterbrühe abgeschieden. Anschließend erfolgt eine Aufkonzentration des Wertstoffes in einer weiteren Aufarbeitungsanlage, z.B. durch Fällung oder in einer Ultrafiltration bzw. Umkehrosmose. Sowohl die Mikrofiltration mit Porengrößen von 0,02 - 10 $\mu$m als auch die Ultrafiltration mit Porengrößen von 0,001 - 0,02 $\mu$m werden mit Hilfe hintereinander geschalteter, sogenannter Modulen durchgeführt. Dabei muß die in den ersten Modul eintretende Fermenterbrühe mit einem ausreichend hohen Druck beaufschlagt werden, damit aufgrund der in den Modulen auftretenden Druckverluste auch im letzten Modul noch eine ausreichend große transmembrane Druckdifferenz zur Stoffabtrennung gewährleistet ist.

Nachteilig bei diesem bekannten Verfahren ist das bei Membranverfahren immer auftretende Problem der sogenannten Konzentrationspolarisation. Durch den Stofffluß an die Membranoberfläche gelangen neben den Wertstoffen auch Stoffe an die Poren der Membran, die diese aufgrund ihrer Größe nicht passieren können. Dadurch bildet sich auf der Membranoberfläche eine komplex zusammengesetzte Deckschicht, die während der Aufkonzentrierung mehr und mehr den Stofffluß, insbesondere des Wertstoffes, durch die Membran blockiert. Dadurch wird die Retention (Rückhaltung der Wertstoffe) erhöht und der Permeatfluß begrenzt. Zur Aufrechterhaltung der Trennleistung des Verfahrens muß die Aufkonzentrierung bei relativ niedrigem Aufkonzentrierungsgrad abgebrochen werden und die Membranflächen müßten einer Reinigung unterzogen werden.

Aus der EP-A-0 137 877 ist ein Verfahren zur Abtrennung biologisch aktiver Materialien durch Querstrom-Ultrafiltration bekannt, bei dem permeatseitig an jedem Modul ein Gegendruck angelegt wird, um in jedem Modul etwa die gleiche transmembrane Druckdifferenz zu gewährleisten. Bei der Ultrafiltration werden jedoch Rückstände von der Lösung getrennt, wobei die Rückstände konzentratseitig auf der Membran verbleiben und nur die Lösung durch die Membran hindurch auf die Permeatseite gelangt. Bei der Mikrofiltration derartiger Fermenterbrühen hingegen werden auf der Konzentratseite der Membran Rückstände (Zellbestandteile) zurückgehalten und durch die Membran hindurch gelangen die Lösung und Wertstoffe (Enzyme) auf die Permeatseite der Membran. Dabei ist es erstrebenswert, daß der Wertstoff nahezu vollständig durch die Membran tritt, während gleichzeitig die Rückstände nahezu vollständig konzentratseitig von der Membran zurückgehalten werden sollen.

Aufgabe der Erfindung ist deshalb die Schaffung einer Lösung, die bei hohem Permeatfluß eine möglichst geringe Retention des Wertstoffes ermöglicht und universell eingesetzt werden kann.

Erfindungsgemäß wird diese Aufgabe gelöst durch die Verwendung mehrerer seriell hintereinander geschalteter und zur Erzeugung einer transmembranen Druckdifferenz zwischen Konzentrat- und Permeatseite permeatseitig mit je Modul unterschiedlichem Gegendruck betriebener Module mit Trennmembran für die Abtrennung von in einer Lösung gelöster Rückstände bei der Querstrom-Mikrofiltration zur Trennung einer Fermenterbrühe in biotechnologisch hergestellte Wertstoffe und Rückstände, wobei die wertstoffe durch die Membran in das Permeat gelangen.

Es hat sich überraschend herausgestellt, daß das für die Ultrafiltration bekannte Gegendruckmembranverfahren auch für die Mikrofiltration geeignet ist, bei der nicht nur Rückstände konzentratseitig zurückgehalten werden sollen, sondern insbesondere der Wertstoff durch die Membran vollständig in das Permeat gelangen soll.

In Ausgestaltung sieht die Erfindung vor, daß der permeatseitige Überdruck gegenüber dem Umgebungsdruck an den Modulen vom Eingangsmodul bis zum Ausgangsmodul derart abgesenkt wird, daß in allen Modulen eine etwa gleichgroße mittlere transmembrane Druckdifferenz zwischen Konzentrat und Permeat aufgebracht wird.

Aufgrund der Druckverluste in den Modulen ist der Eintrittsdruck in den ersten Modul auf der Konzentratseite gegenüber dem Austrittsdruck des letzten Moduls wesentlich größer. Deshalb wird die transmembrane Druckdifferenz bei konventionellen Membrantrennverfahren von Modul zu Modul abgebaut. Dadurch ist die transmembrane Druckdifferenz wenigstens im ersten Modul so hoch, daß die Retention des Wertstoffes groß wird. Durch die Ausgestaltung der Erfindung wird dagegen eine konstante transmembrane Druckdifferenz über alle Modulen erreicht, die eine konstante, geringe Retention gewährleistet.

Erfindungsgemäß kann vorgesehen sein, daß im letzten Modul permeatseitig ein Unterdruck gegenüber dem Umgebungsdruck angelegt wird. Je nach Einsatzfall ist es zur Erzielung einer gleichbleibenden transmembranen Druckdifferenz über alle Module empfehlenswert, in der letzten Stufe einen permeatseitigen Unterdruck anzulegen.

Die Erfindung sieht weiterhin vor, daß die Mikrofiltration mit Überströmgeschwindigkeiten von mehr als 4 m/s, insbesondere von 5 - 7 m/s, betrieben wird. Es hat sich herausgestellt, daß diese Überströmgeschwindigkeiten besonders günstig sind.

Ferner ist vorgesehen, daß anorganische Materialien, wie Aluminiumoxid, Siliciumcarbid oder Zirkoniumdioxid auf einem Träger, als Membranwerkstoff eingesetzt werden. Diese Membranen haben den Vorteil, daß sie durch Dampfeinwirkung sterilisiert werden können, keine Membrankompaktion auftritt und sie keiner Abrasion unterliegen. Aber auch entsprechende organische Membranen lassen sich gemäß der Erfindung betreiben.

In weiterer Ausgestaltung ist vorgesehen, bei Einsatz der Mikrofiltration das Permeat bei einer nachgeschalteten Ultrafiltration zur Verdünnung der Fermenterbrühe sowie als Diafiltrationsflüssigkeit einzusetzen. Auch kann eine Salzlösung verwendet werden.

Die Erfindung ist nachstehend anhand der Zeichnung beispielsweise näher erläutert. Diese zeigt in schematischer Darstellung in

Fig. 1    das Prinzip der Querstrom-Membranfiltration für die Abtrennung von Wertstoffen aus Kultur- bzw. Fermenterlösungen und in

Fig. 2    ein vereinfachtes Verfahrensfließbild einer Querstrom-Membranfiltrationsanlage zur Durchführung des erfindungsgemäßen Verfahrens.

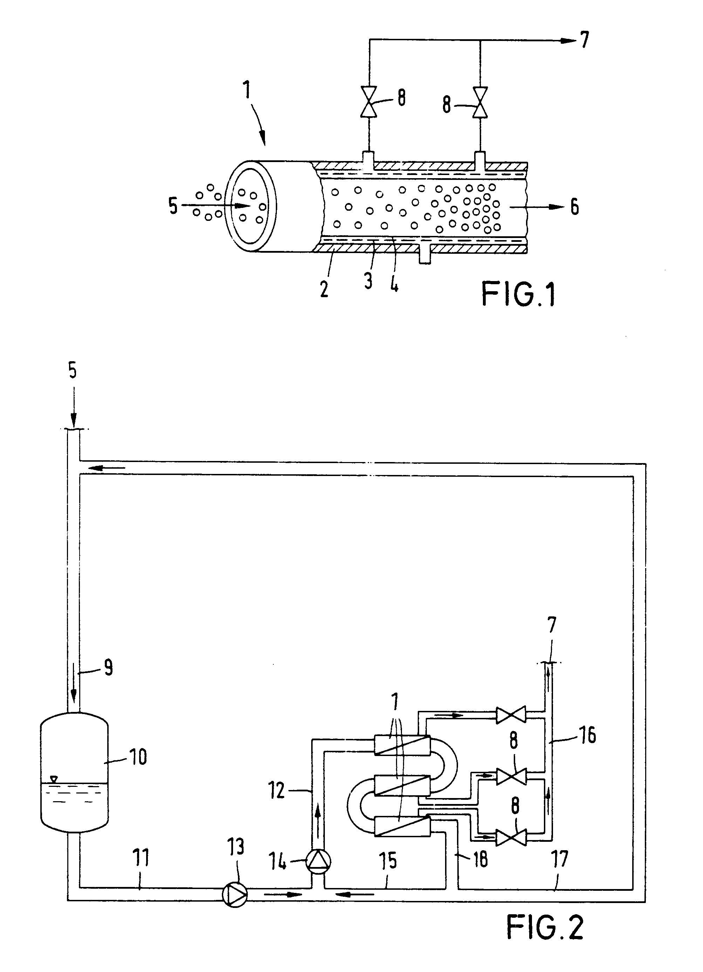

Bei dem in Fig. 1 dargestellten Verfahrensprinzip zur Abtrennung von biotechnologisch hergestellten Wertstoffen aus einer Zellsuspension (Fermenterbrühe, gegebenenfalls nach Zelldesintegration) durch Querstrom-Membranfiltration findet ein rohrförmiges Trennmodul 1 Verwendung. Das Trennmodul 1 besteht aus einem Stützrohr 2, in das ein Trägerrohr 3 eingelassen ist, auf dessen Mantelinnenfläche eine trennaktive Membranschicht 4 aufgebracht ist. Das rohrförmige Teilmodul 1 weist im Beispiel eine Länge von etwa 1,2 m auf. Bei besonders stabilen Trägerrohren kannt das Stützrohr 2 entfallen.

Die Kultur- bzw. Fermenterlösung 5 wird in Pfeilrichtung mit Strömungsgeschwindigkeiten von etwa 5 - 7 m/s über die trennaktive Membranschicht 4 geführt, wobei die Wertstoffe die Membranschicht 4 in radialer Richtung durchdringen, während Zellen, Zellbruchstücke und Feststoffe von der Membranschicht 4 zurückgehalten werden.

Es bildet sich ein flüssiges Konzentrat 6, welches die Zellen, Zellbruchstücke und Feststoffe enthält sowie ein flüssiges Permeat 7, worin sich der abgetrennte Wertstoff befindet. Die treibende Kraft zur Trennung von Konzentrat 6 und Permeat 7 stellt eine transmembrane Druckdifferenz dar. Diese transmembrane Druckdifferenz wird durch einen Überdruck auf der Konzentratseite gegenüber der Permeatseite erreicht. Um in allen Modulen 1 eine gleichbleibende transmembrane Druckdifferenz aufbringen zu können, ist auf der Permeatseite ein Überdruck gegenüber der Umgebung einstellbar, der über Drosseln 8 zur Ableitung des Permeats 7 abgebaut wird.

Die rohrförmigen Trennmodule 1 stellen den wesentlichen Bestandteil des in Fig. 2 dargestellten, vereinfachten Verfahrensfließbildes einer Querstrom-Mikrofiltrationsanlage dar. Die Fermenterlösung 5 wird über eine Rohrleitung 9 in einen temperierbaren Vorlagebehälter 10 geleitet oder direkt dem Fermenter entnommen, aus dem sie über Rohrleitungen 11, 12 mit Hilfe einer Speisepumpe 13 in der Leitung 11 und einer Zirkulationspumpe 14 in der Leitung 12 in die rohrförmigen Trennmodule 1 gelangt. Drei hintereinander geschaltete Trennmodule 1 bilden hier eine Stufe. In jedem der drei rohrförmigen Trennmodule 1 wird die Kulturbzw. Fermenterlösung 5 in das Konzentrat 6 und das Permeat 7 getrennt, wobei das Konzentrat 6 über Rohrleitungen in das jeweils nächste Modul 1 gelangt, bzw. aus dem letzten Modul über Rohrleitungen 18 und 15 zum größten Teil über die Zirkulationspumpe 14 zurückgeführt wird, während das Permeat 7 aus jedem Trennmodul 1 nach Druckentspannung in eine Abflußleitung 16 abfließt. Ein kleinerer Teil des Konzentrats 6 gelangt bei batch-Betrieb über eine Abzweigleitung 17 und die Leitung 9 in den Behälter 10 oder Fermenter zurück. Bei einem kontinuierlich durchgeführten Prozeß wird das Konzentrat 6 der Leitung 17 direkt entnommen. In Fig. 2 ist eine einstufige Ausführung der Trennanlage wiedergegeben. In der Technik arbeitet man normalerweise mit bis zu 10 Trennstufen.

In einer typischen Ausführungsform der Erfindung werden drei Module 1 mit einer Länge von jeweils 1,2 m hintereinander geschaltet und so betrieben, daß der Ausgangsdruck des ersten Moduls auf der Konzentratseite dem Eingangsdruck des zweiten Moduls usw. entspricht. Der Druckabfall pro Modul beträgt dabei 1,8 bar, insgesamt also 5,4 bar. Die Fermenterlösung 5 gelangt mit einem Eintrittsdruck von 6,1 bar gegenüber der Umgebung in den ersten Modul 1. Der Ausgangsüberdruck im letzten Modul beträgt demnach 0,7 bar. Um in allen Stufen eine gleichbleibende mittlere transmembrane Druckdifferenz zu erzielen, wird auf der Permeatseite 7 von Stufe zu Stufe ein abnehmender Überdruck gegenüber der Umgebung angelegt. Dabei beträgt der Überdruck auf der Permeatseite beim ersten Modul 3,6 bar, beim zweiten 1,8 bar und beim dritten 0 bar. Damit ergibt sich in allen Stufen eine konstante, mittlere transmembrane Differenz von 1,6 bar. Das unter Druck stehende Permeat 7 wird in den Drosseln 8 entspannt, ehe es in die Abflußleitung 16 gelangt. Das Verfahrensprinzip kann auf beliebig viele hintereinander geschaltete Module angewandt werden, von zwei Modulen an aufwärts.

In weiteren Ausführungsformen ist vorgesehen, das Permeat 7 über die Abflußleitung 16 in eine Aufbereitungsanlage, z.B. in eine Fällstufe oder Ultrafiltrationsanlage zur Anreicherung der Wertstoffe zu führen. Auch die Ultrafiltrationsanlage kann, falls erforderlich, nach derselben Gegendrucktechnik wie bei der Querstrom-Mikrofiltration, d.h also mit Aufbringen eines stufenabhängigen, permeatseitigen Überdruckes gegenüber der Umgebung, durchgeführt werden.

Auch kann vorgesehen sein, daß das Permeat der Ultrafiltration zur Verdünnung der Fermenterbrühe sowie als Diafiltrationsflüssigkeit eingesetzt wird. Bewährt hat sich auch die Vorverdünnung und Diafiltration mit Salzlösungen, insbesondere mit solchen Salzen, die die Proteine stabilisieren.

Das beschriebene Verfahren eignet sich auch dann zur Gewinnung von Wertstoffen, wenn diese von den sie erzeugenden Mikroorganismen nicht in das Fermentationsmedium ausgeschieden werden, sondern erst nach Zerstörung der Zellen mit mechanischen oder chemischen (enzymatischen) Mitteln gewonnen werden können.

Die Erfindung ist nicht auf das in der Zeichnung dargestellte Ausführungsbeispiel beschränkt. Die Module können anders ausgeführt sein, beispielsweise als Plattenmodul oder multi-channel-Einheit, und anders in der Länge unterteilt werden und dgl. mehr.

**Patentansprüche**

1. Verwendung mehrerer seriell hintereinander geschalteter und zur Erzeugung einer transmembranen Druckdifferenz zwischen Konzentrat- und Permeatseite permeatseitig mit je Modul unterschiedlichem Gegendruck betriebener Module mit Trennmembran für die Abtrennung von in einer Lösung gelöster Rückstände bei der Querstrom-Mikrofiltration zur Trennung einer Fermenterbrühe in biotechnologisch hergestellte Wertstoffe und Rückstände, wobei die Wertstoffe durch die Membran in das Permeat gelangen.

2. Verwendung nach Anspruch 1,
   dadurch gekennzeichnet,
   daß der permeatseitige Gegendruck vom Eingangsmodul bis zum Ausgangsmodul derart abgesenkt wird, daß in allen Modulen eine etwa gleichgroße, mittlere transmembrane Druckdifferenz zwischen Konzentrat und Permeat aufgebracht wird.

3. Verwendung nach Anspruch 1 oder 2,
   dadurch gekennzeichnet,
   daß im letzten Modul permeatseitig ein Unterdruck gegenüber dem Umgebungsdruck angelegt wird.

4. Verwendung nach Anspruch 1 oder einem der folgenden,
   dadurch gekennzeichnet,
   daß die Mikrofiltration mit Überströmgeschwindigkeiten von mehr als 4 m/s, insbesondere von 5 - 7 m/s betrieben wird.

5. Verwendung nach Anspruch 1 oder einem der folgenden,
   dadurch gekennzeichnet,
   daß anorganische Materialien, wie Aluminiumoxid, Siliciumcarbid oder Zirkoniumdioxid auf einem Träger als Membranwerkstoff eingesetzt werden.

6. Verwendung nach Anspruch 1 oder einem der folgenden,
   dadurch gekennzeichnet,
   daß das Permeat bei einer nachgeschalteten Ultrafiltration zur Verdünnung der Fermenterbrühe sowie als Diafiltrationsflüssigkeit eingesetzt wird.

7. Verwendung nach Anspruch 6,
   dadurch gekennzeichnet,
   daß zur Verdünnung der Fermenterbrühe sowie als Diafiltrationsflüssigkeit eine Salzlösung verwendet wird.

**Claims**

1. The use of several modules serially arranged one behind the other and each operated with a different counterpressure on the permeate side to produce a transmembranal pressure difference between the concentrate side and the permeate side and comprising a separation membrane for removing residues dissolved in a solution during crossflow microfiltration for separating a fermenter broth into biotechnologically produced valuable materials and residues, the valuable materials entering the permeate through the membrane.

2. The use claimed in claim 1, characterized in that the counterpressure on the permeate side is reduced from the entry module to the exit module so that an equal mean transmembranal pressure difference between concentrate and permeate is applied in all the modules.

3. The use claimed in claim 1 or 2, characterized in that a reduced pressure relative to the ambient pressure is applied on the permeate side in the last module.

4.  The use claimed in claim 1 or any of the following claims, characterized in that microfiltration is carried out at crossflow rates of more than 4 m/s and, more particularly, in the range from 5 to 7 m/s.

5.  The use claimed in claim 1 or any of the following claims, characterized in that inorganic materials, such as aluminium oxide, silicon carbide or zirconium dioxide on a support are used as the membrane material.

6.  The use claimed in claim 1 or any of the following claims, characterized in that the permeate is used in a following ultrafiltration to dilute the fermenter broth and as diafiltration liquid.

7.  The use claimed in claim 6, characterized in that a salt solution is used to dilute the fermenter broth and as diafiltration liquid.

**Revendications**

1.  Utilisation de plusieurs modules, munis de membrane de séparation, branchés en série les uns derrière les autres et commandés du côté perméat par une contre pression différente à chaque module de façon à obtenir une différence de pression à travers la membrane entre côté concentré et côté perméat, modules destinés à la séparation de résidus dissous dans une solution en utilisant la micro filtration tangentielle apte à diviser un milieu de fermentation en matières de valeur préparées par des procédés biotechnologiques et en résidus, les matières de valeur arrivant à travers la membrane dans le perméat.

2.  Utilisation selon la revendication 1, caractérisée en ce que la contre pression du côté perméat est diminuée depuis le module d'entrée jusqu'au module de sortie, de manière à appliquer dans tous les modules une différence de pression moyenne sensiblement de même grandeur à travers la membrane entre concentré et perméat.

3.  Utilisation selon les revendications 1 ou 2, caractérisée en ce que dans le dernier module du côté perméat est appliquée une sous pression par rapport à la pression environnante.

4.  Utilisation selon la revendication 1 ou une des revendications suivantes, caractérisée en ce que la micro filtration est opérée avec des vitesses de courant de passage supérieures à 4 m/s, en particulier allant de 5 à 7 m/s.

5.  Utilisation selon la revendication 1 ou une des revendications suivantes, caractérisée en ce que des matières minérales, comme oxyde d'aluminium, carbure de silicium ou dioxyde de zirconium sont utilisées comme matériau de membrane sur un support.

6.  Utilisation selon la revendication 1 ou une des revendications suivantes, caractérisée en ce que le perméat est mis en oeuvre lors d'une ultra filtration introduite à la suite pour diluer le milieu de fermentation ainsi que comme liquide de dia filtration.

7.  Utilisation selon la revendication 6, caractérisée en ce qu'on utilise une solution de sels pour diluer le milieu de fermentation ainsi que comme liquide de dia filtration.

FIG.1

FIG.2